# EUROPÄISCHE PATENTSCHRIFT

(11) **EP 2 838 594 B1**
(45) Veröffentlichungstag und Bekanntmachung des Hinweises auf die Patenterteilung: **20.09.2017**
(21) Anmeldenummer: 13715206.2
(22) Anmeldetag: 09.04.2013
(51) Int. Cl.: A61M 15/00

(54) **VORRICHTUNG ZUM INHALIEREN PULVERFÖRMIGER SUBSTANZEN**
DEVICE FOR INHALING POWDERY SUBSTANCES
DISPOSITIF POUR L'INHALATION DE SUBSTANCES PULVÉRULENTES

(30) Priorität: 20.04.2012 DE 102012103482
(43) Veröffentlichungstag der Anmeldung: 25.02.2015
(73) Patentinhaber: Von Schuckmann, Alfred, 47627 Kevelaer (DE)
(72) Erfinder: Von Schuckmann, Alfred, 47627 Kevelaer (DE)
(74) Vertreter: Müller, Enno
(86) Internationale Anmeldenummer: PCT/EP2013/057344
(87) Internationale Veröffentlichungsnummer: WO 2013/156339

(56) Entgegenhaltungen:
- WO-A2-2011/039307
- DE-A1-102006 010 089
- US-B1- 6 606 992

## Beschreibung

Die Erfindung betrifft eine Vorrichtung zum Inhalieren pulverförmiger Substanzen nach den Merkmalen des Oberbegriffes des Anspruches 1.

Vorrichtungen der in Rede stehenden Art sind bekannt, so weiter auch unter dem Begriff "Kapselinhalatoren". Diese dienen vornehmlich der Inhalation medikamentöser, pulverförmiger Massen, die weiter portioniert in Kapseln vorliegen. In der Regel bestehen derartige Kapseln aus zwei Teilen, nämlich einem Kapselkörper und einer Kapselkappe, die teleskopartig ineinandergeschoben sind, wobei insbesondere zur Aufnahme inhalationsfähiger Substanzen Kapseln aus einem, sehr geringe Wasseraufnahmefähigkeiten aufweisenden Kunststoff bestehen, alternativ aus Gelatine. Aus der DE 10 2010 016549 A1 ist eine Vorrichtung der in Rede stehenden Art bekannt. Bei dieser wird die, die zu inhalierende Substanz aufnehmende Kapsel in eine Kapselkammer mittels eines manuell durch den Benutzer zu verlagernden Schiebers eingelegt. Dieser Schieber steht in der Kapselaufnahmestellung über die Schmalseitenwand des Flachgehäuses vor. Durch manuelle Verlagerung des Schiebers in Richtung auf das Gehäuseinnere wird die Kapsel in eine Inhalations-Vorbereitungsstellung verlagert, in welcher Stellung die die Kapsel aufnehmende Kapselkammer in axialer Verlängerung zu dem Mundstückkanal liegt. In dem die Kapselkammer aufweisenden Schieber ist ein weiterer in derselben Verlagerungsrichtung bewegbarer, drucktastenartiger Schieber angeordnet, dessen Betätigungsfläche zur Beaufschlagung über die Schmalseitenwand des Gehäuses auch in der Nichtbenutzungsstellung der Vorrichtung vorsteht. Durch weitere Verlagerung des drucktastenartigen Schiebers wird die Kapselwandung mittels an diesem drucktastenartigen Schieber festgelegten Nadeln durchstochen, dies unter Überwindung der die Nadeln und den drucktastenartigen Schieber in eine Nichtbelastungsstellung zurückdrängenden Federkraft. Zur Inhalation sind die Nadeln und der drucktastenartige Schieber wieder zurück in ihre federbelastete Grundstellung verlagert.

Bei einer aus der DE 10 2006 010 089 A1 bekannten Vorrichtung ist die Aufnahme für eine Kapsel seitlich einer Breitseite der Vorrichtung ausklappbar vorgesehen. Die Nadeln zum Durchstechen sind unabhängig von der Aufnahme vorgesehen und in Vertikalrichtung der Vorrichtung zu betätigen. Weiter ist aus der WO2011/039307 A2 eine derartige Vorrichtung bekannt, bei welcher die Nadeln in einer seitlichen Drucktaste befestigt sind, die Aufnahme jedoch unabhängig hiervon von oben in der Vorrichtung zu beschicken ist.

Ausgehend von dem vorgenannten Stand der Technik beschäftigt sich die Erfindung mit der Aufgabenstellung eine Vorrichtung der genannten Art anzugeben, die hinsichtlich einer benutzerfreundlichen Bedienung und Nutzung der Vorrichtung vorteilhaft ist.

Dieser Aufgabe ist beim Gegenstand des Anspruches 1 gelöst, wobei darauf abgestellt ist, dass die Aufnahme um eine Achse als begrenzt ausschwenkbarer Teil einer Schmalseitenwand des Flachgehäuses ausgebildet ist und ein Teilbereich einer Schmalseitenwand-Ebene als abgefederte Drucktaste für Nadellager, in welchem die Nadeln gefasst sind, ausgebildet ist, derart, dass sich der Teilbereich bis maximal in die Ebene der Schmalseitenwand zurückstellt und dass die Drucktaste in dem ausschwenkbaren Gehäuseteil anschlagbegrenzt geführt ist. Zufolge dieser Ausgestaltung ist eine insbesondere in der unbelasteten Grundstellung, d.h. weiter insbesondere in der Nichtbenutzungsstellung ringsum geschlossene Bauform der Vorrichtung gegeben. In dieser Nichtbenutzungsstellung liegt der die Aufnahme aufweisende Teil eingeschwenkt in dem Flachgehäuse ein, wobei dieser Teil zumindest einen Abschnitt der Schmalseitenwand insbesondere in der Nichtbenutzungsstellung der Vorrichtung ausbildet, welche Schmalseitenwand bevorzugt parallel verläuft zu einer gegenüberliegenden Schmalseitenwand. Die insbesondere schieberartig verlagerbare, abgefederte Drucktaste zur Verlagerung der Nadeln in eine, die Kapsel in der Kapselkammer durchstoßende Stellung steht in der insbesondere durch den Benutzer nicht belasteten Grundstellung nicht über die Ebene der Schmalseitenwand hinaus. Vielmehr stellt sich der, die Drucktaste ausbildende Teilbereich lediglich maximal bis in die Ebene der Schmalseitenwand zurück. Die hierdurch erreichte, bevorzugt insgesamt geschlossene Bauform der Vorrichtung, weiter insbesondere in der Stellung, in welcher das Mundstück, wie weiter bevorzugt, durch eine Kappe verschlossen ist, bietet insbesondere Vorteile beim Tragen der Vorrichtung in einer Tasche oder dergleichen, beispielsweise in einer Jacken- oder Hosentasche. In dieser Aufbewahrungsstellung der Vorrichtung sind bevorzugt alle lufttechnisch relevanten Öffnungen der Vorrichtung durch Gehäuseabschnitte und/ oder durch eine insbesondere das Mundstück zugleich überdeckenden Kappe verschlossen, womit einem Eindringen von beispielsweise Staub- oder Schmutzpartikeln, insbesondere in den Lufteinlass oder auch darüber hinaus in die Kapselkammer entgegengewirkt ist. Die Vorrichtung weist in bevorzugter Ausgestaltung ein handliches, etwa Handteller-großes Flachgehäuse auf, welches eine günstige Bedienung der Vorrichtung erlaubt. Hierbei ist in Bezug auf einen Querschnitt ein Breiten-/Tiefenverhältnis des Flachgehäuses von bevorzugt 2 : 1 gegeben. Die Höhe der Vorrichtung inklusive einer bevorzugt vorgesehenen, das Mundstück überdeckenden Kappe beträgt bevorzugt dem 3- bis 5-Fachen der quer hierzu betrachteten Breite. Das die Aufnahme ausformende, begrenzt nach außen ausschwenkbare Teil ist weiter bevorzugt schwenkbar um eine quer zur Breitenrichtung des Flachgehäuses verlaufende Schwenkachse. Ist die das Mundstück in der Nichtbenutzungsstellung der Vorrichtung überdeckende Kappe, wie weiter bevorzugt, gleichfalls unverlierbar an dem Flachgehäuse schwenkbar angeordnet, so erstreckt sich deren Schwenkachse in bevorzugter Ausgestaltung parallel zur Schwenkachse des die Aufnahme aufweisenden Teils.

Weiter bevorzugt ist der Einsteckquerschnitt der Kapselkammer in der Öffnungsstellung der Aufnahme in spitzwinkliger Anschlagstellung gestoppt. Entsprechend ist bevorzugt der die Aufnahme bildende Teil der Schmalseitenwand des Flachgehäuses in eine spitzwinklige Stellung relativ zu einer parallel zur Schmalseitenwand des Flachgehäuses verlaufenden Ebene anstellbar, um so die Kapselkammer zur Bestückung mit einer Kapsel freizulegen. Diese Ausschwenkstellung ist, wie weiter bevorzugt auch die eingeschwenkte Grundstellung, anschlagbegrenzt. Hierbei wird in der ausgeschwenkten Stellung bevorzugt ein spitzer Winkel insbesondere einer die Kapselkammer in Längsrichtung durchsetzenden Mittelachse relativ zu einer durch die Schmalseitenwand in der Nichtbenutzungsstellung definierten Ebene von 15 bis 60 Grad, weiter bevorzugt 30 bis 45 Grad, eingenommen. Die Kapselkammer liegt hiernach in einer für den Benutzer günstig zugänglichen Stellung, in welcher eine entsprechende Bestückung mit einer neuen Kapsel beziehungsweise die Entnahme einer entleerten Kapsel vorgenommen werden kann.

Auch ist bevorzugt vorgesehen, dass die Schmalseitenwand mitsamt einem nahezu halben, kastenförmigen Gehäuseteil ausschwenkt, einschließlich eines Bodenteils, welches den Schwenkbolzen besitzt. Der Schwenkbolzen wirkt hierbei bevorzugt zusammen mit dem weiteren halben, insbesondere kastenförmigen Gehäuseteil, was relativ zu dem ausschwenkbaren Teil feststeht. Das ausschwenkbare Teil ist hierbei weiter bevorzugt schubladenartig in das im Wesentlichen feststehende Gehäuseteil einschwenkbar, weiter bevorzugt entsprechend beidseitig flankiert von Gehäusewandungen des im Wesentlichen feststehenden Gehäuseteils.

Die abgefederte Drucktaste ist zudem bevorzugt formschlussgeführt in eine Stellung, in der die Drucktaste mit dem restlichen Bereich der Schmalseitenwand in einer Ebene liegt. Hierdurch ist in vorteilhafter Weise insgesamt in der Grundstellung der Vorrichtung, d.h. in der eingeschwenkten Stellung des einen Gehäuseteils und in der von außen nicht belasteten Grundstellung der Drucktaste eine im Wesentlichen ebene Ausgestaltung der diesbezüglichen Schmalseitenwand erreicht. Dies ist insbesondere erreicht durch eine anschlagbegrenzte Formschlussführung der Drucktaste im Bereich des ausschwenkbaren Gehäuseteils. Die auf die Drucktaste einwirkende Feder drängt hierbei die Drucktaste gegen den vorgesehenen Anschlag der Formschlussführung. Auch ist hierdurch eine gezielte Verlagerung der Drucktaste mit den daran angeordneten Nadeln in Richtung auf die Kapselwandung-Durchstechstellung erreichbar.

Die vor- und nachstehend angegebenen Bereiche bzw. Wertebereiche oder Mehrfachbereiche schließen hinsichtlich der Offenbarung auch sämtliche Zwischenwerte ein, insbesondere in 1/10- Schritten der jeweiligen Dimension, ggf. also auch dimensionslos, insbesondere 1,01-Fach etc. einerseits zur Eingrenzung der genannten Bereichsgrenzen von unten und/oder oben, alternativ oder ergänzend aber auch im Hinblick auf die Offenbarung eines oder mehrerer singulärer Werte aus dem jeweils angegebenen Bereich.

Nachstehend ist die Erfindung anhand der beigefügten Zeichnung erläutert, die aber lediglich ein Ausführungsbeispiel darstellt. Auf der Zeichnung zeigt:
- Fig. 1: die Vorrichtung in perspektivischer Darstellung, eine deckelverschlossene Nichtbenutzungsstellung betreffend;

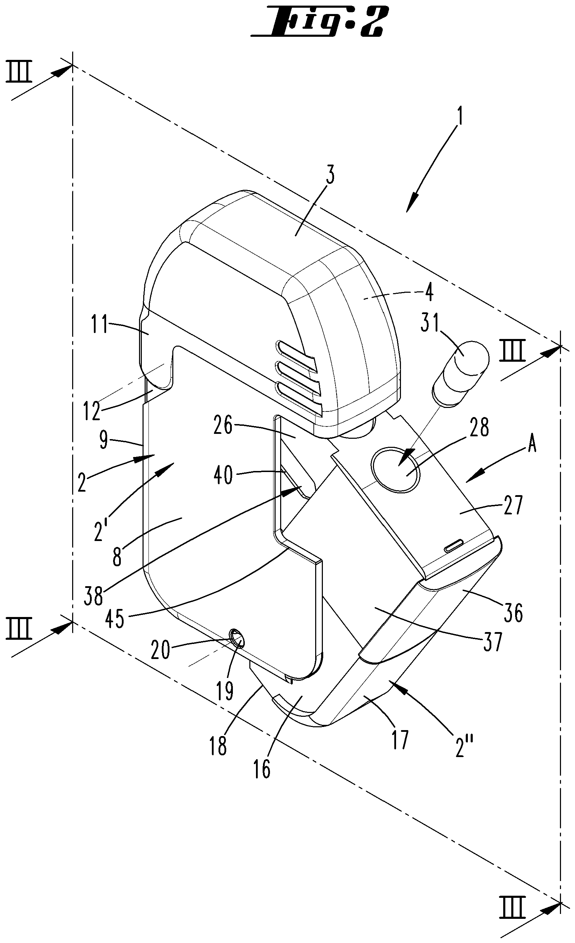

- Fig. 2: die Vorrichtung in perspektivischer Darstellung gemäß Fig. 1, jedoch die Gebrauchs-Vorbereitungsstellung betreffend;
- Fig. 3: den Schnitt gemäß der Schnittebene III in Fig. 2;
- Fig. 4: eine der Fig. 3 entsprechende Schnittdarstellung, die Verlagerungsstellung eines die Kapselkammer aufweisenden Gehäuseteils in die Inhalierposition betreffend;
- Fig. 5: eine Folgedarstellung der Fig. 4 nach Abschwenken einer, ein Mundstück überdeckenden Kappe und Freilegen einer Lufteinströmöffnung und im Zuge des Durchstechens einer in der Kapselkammer aufgenommenen Kapsel durch Schiebeverlagerung eines Schmalseitenwandabschnittes;

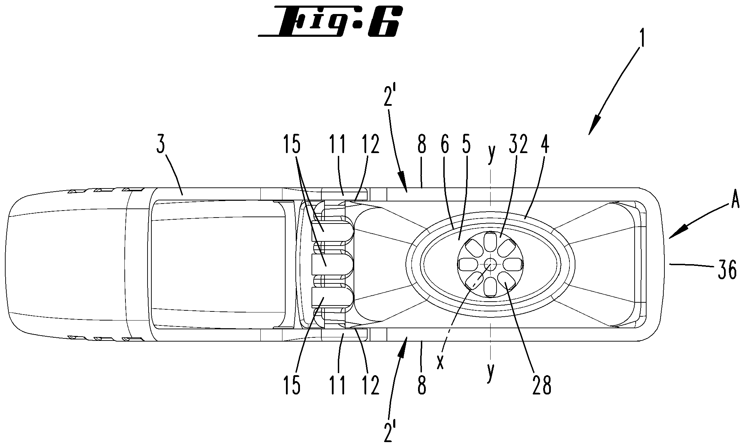

- Fig. 6: die Draufsicht auf die Vorrichtung bei abgeschwenkter Kappe;

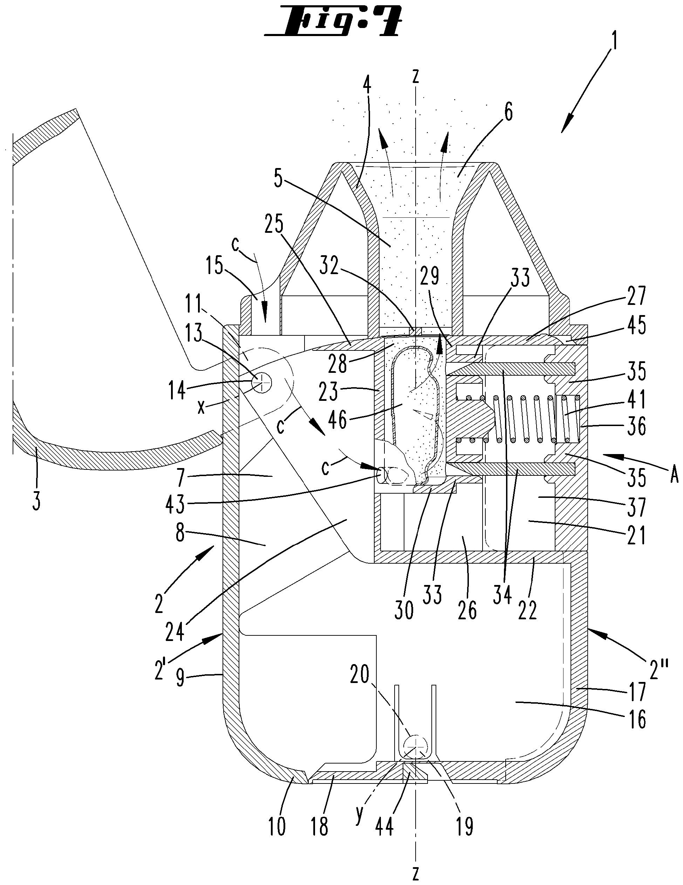

- Fig. 7: eine Folgedarstellung zu Fig. 5 nach Rückverlagerung des die Nadeln tragenden Teils der Schmalseitenwand, betreffend den Inhalationsvorgang;

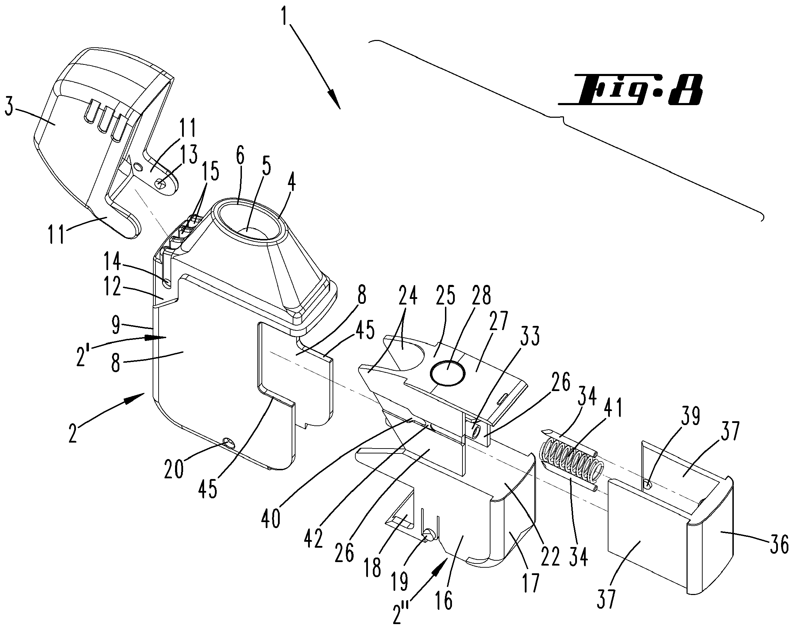

- Fig. 8: eine perspektivische Explosionsdarstellung der Vorrichtung.

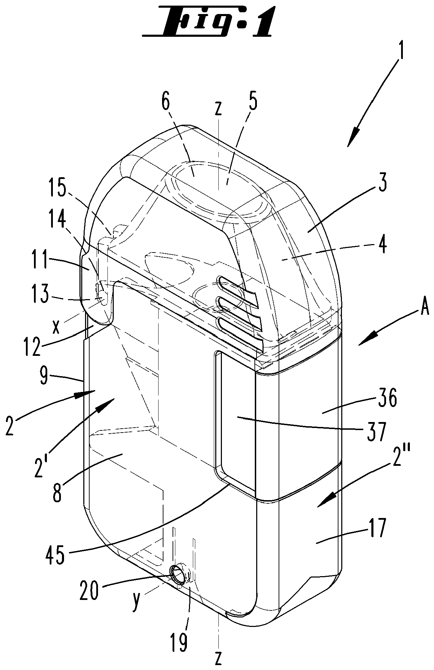

Dargestellt und beschrieben ist zunächst mit Bezug zu Figur 1 eine Vorrichtung 1 zum Inhalieren pulverförmiger Substanzen in Art eines Kapselinhalators, welche Vorrichtung 1 als bequem mitführbares Taschengerät realisiert ist.

Die Vorrichtung 1 weist ein im Wesentlichen zweigeteiltes Flachgehäuse 2 auf, so mit einem insbesondere durch Ergreifen mit der Hand quasi feststehendes Gehäuseteil 2' und einem relativ zu diesem Gehäuseteil 2' schwenkbar verlagerbaren Gehäuseteil 2".

An dem Gehäuse 2, insbesondere an dem feststehenden Gehäuseteil 2' ist eine deckelartige Kappe 3 anscharniert.

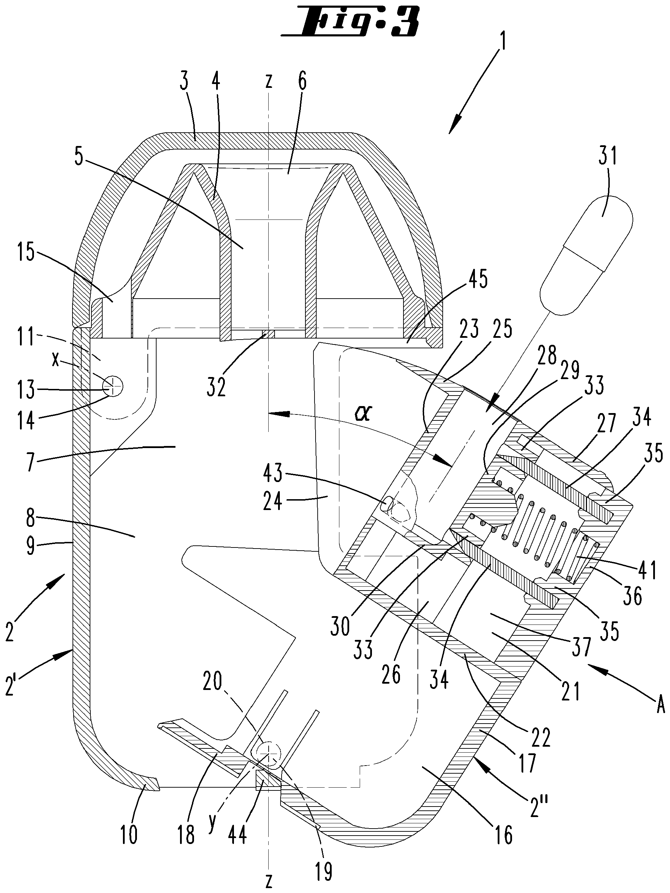

In der Kappenverschlussstellung, weiter in einer Nichtbenutzungsstellung gemäß der Fig. 1, weist die Vorrichtung 1 ein Breiten-Höhenverhältnis von etwa 1 : 2 auf und eine senkrecht zur Höhenerstreckung, d.h. weiter eine senkrecht zu der Bildebene beispielsweise in Figur 3 betrachtete Tiefe, die etwa dem halben Breitenerstreckungsmaß des Gehäuses 2 entspricht. Die Teile der Vorrichtung 1 sind bevorzugt als Kunststoff-Spritzteile realisiert.

Das Gehäuse 2, insbesondere das feststehende Gehäuseteil 2' formt zunächst ein Mundstück 4 aus, welches in Breitenrichtung des Gehäuses 2 betrachtet etwa mittig angeordnet ist und in Höhenrichtung des Gehäuses 2 sich gegenüber beidseitig benachbarten Gehäusewandungen des feststehenden Gehäuseteiles 2' erhebt, so dass das Mundstück 4 bequem lippenumschlossen werden kann.

In dem Mundstück 4 mündet ein sich mit Bezug auf die Darstellungen vertikal erstreckender Mundstückkanal 5 im Bereich eines nach außen offenen Mundstückausganges 6. Der Mundstückkanal 5 erstreckt sich hierbei, ausgehend von dem Mundstückausgang 6, bevorzugt annähernd über ein Drittel der vertikalen Höhe der Vorrichtung 1 und endet dem Mundstückausgang 6 gegenüberliegend frei in einem in dem Gehäuse 2 ausgebildeten Raum 7. Letzterer ist im Wesentlichen seitlich begrenzt durch die Breitseitenwand 8 des feststehenden Gehäuseteiles 2' und der die Breitseitenwände 8 einseitig endseitig begrenzenden Schmalseitenwand 9. Letztere geht gegenüberliegend zum Mundstück 4 bevorzugt in einen Bodenteilabschnitt 10 über.

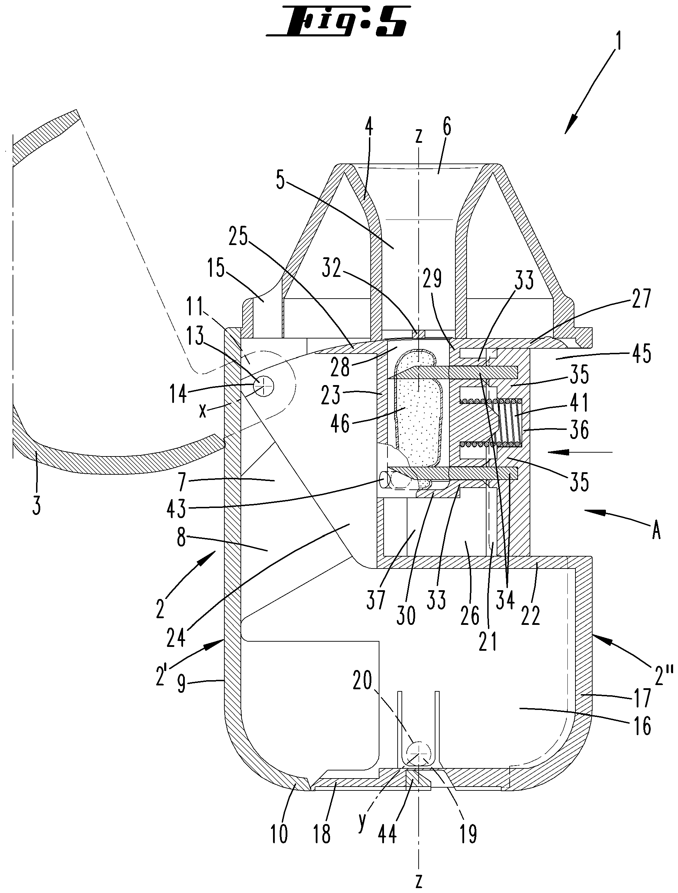

Die Kappe 3 ist zugewandt der Schmalseitenwand 9 an dem feststehenden Gehäuseteil 2' anscharniert. Hierzu weist die Kappe 2 zwei gegenüberliegende, sich im Wesentlichen nach vertikal unten erstreckende Laschen 11 auf, die in entsprechend zugeordnete Oberflächenvertiefungen 12 der Breitseitenwände 8 eingreifen. Wandungsinnenseitig, d.h. der jeweiligen Breitseitenwand 8 zugewandt, ist die Lasche 11 mit einem Zapfen 12 versehen, zum Eingriff in eine entsprechende, bevorzugt sacklochartige Zapfenaufnahme 14. Entsprechend erstreckt sich die Schwenkachse x der Kappe 3 in Tiefenrichtung des Gehäuses 2, wobei bevorzugt ein Abschwenken der Kappe 3 nach außen bis in eine anschlagbegrenzte Stellung ermöglicht ist, in welcher eine Kappenwandung gegen die Außenfläche der zugewandten Schmalseitenwand 9 tritt (vgl. Fig. 5).

Weiter zugewandt der Schmalseitenwand 9 des feststehenden Gehäuseteiles 2' weist die Vorrichtung 1 eine Lufteinlassöffnung 15 auf. Diese ist - in Breitenrichtung des Gehäuses 2 betrachtet - benachbart und axial versetzt zum Mundstück 4, dies etwa um das halbe axiale Erstreckungsmaß des Mundstückkanals 5 in Richtung auf die Schmalseitenwand 9 positioniert. Die Lufteinlassöffnung 15 geht über in den vorbeschriebenen Raum 7.

In der Kappenverschlussstellung gemäß Fig. 1 überfängt die Kappe 3 sowohl das Mundstück 4 mit dem Mundstückausgang 6 als auch die Lufteinlassöffnung 15. Entsprechend können in der Nichtbenutzungsstellung der Vorrichtung 1 insbesondere durch das Mundstück 4 und/oder die Lufteinlassöffnung 15 keine Verschmutzungen in das Innere der Vorrichtung 1 treten.

Das feststehende Gehäuseteil 2' bildet einen im Wesentlichen halben, kastenförmigen Gehäuseabschnitt, welcher sich insbesondere zu der der Schmalseitenwand 9 abgewandten Seite und weiter bevorzugt im Wesentlichen auch in Richtung des Gehäusebodens hin öffnet. Der dem festen Gehäuseteil 2' gegenüber schwenkbare Gehäuseteil 2" formt bevorzugt den weiteren halben, kastenförmigen Gehäuseabschnitt aus, hierbei insbesondere aufweisend zwei Breitseitenwände 16 und eine die beiden Breitseitenwände 16 verbindende Schmalseitenwand 17.

Der Abstand der wie auch beim Gehäuseteil 2' parallel zueinander verlaufenden Breitseitenwände 16 entspricht - mit Bezug auf deren nach außen weisenden Flächen - im Wesentlichen dem lichten Abstand der aufeinander zu weisenden Innenflächen der Breitseitenwände 8 des Gehäuseteiles 2'. Die mit Bezug auf die Darstellungen in Tiefenrichtung betrachtete Erstreckung der Schmalseitenwand 17 entspricht bevorzugt im Wesentlichen der der gegenüberliegenden, in der Nichtbenutzungsstellung der Vorrichtung 1 parallel verlaufenden Schmalseitenwand 9 des Gehäuseteiles 2', womit die an dem schwenkbaren Gehäuseteil 2" ausgebildete Schmalseitenwand 17 mit den zugeordneten Vertikalrandkanten der Breitseitenwände 8 des feststehenden Gehäuseteiles 2' anschlagbegrenzend zusammenwirken.

Darüber hinaus weist der schwenkbare Gehäuseteil 2' ein angeformtes Bodenteil 18 auf, welches in der Nichtbenutzungsstellung der Vorrichtung 1 zusammen mit dem Bodenteilabschnitt 10 des festen Gehäuseteiles 2' die Vorrichtung 1 beziehungsweise das Gehäuse 2 nach unten abschließt.

Im Bereich des Bodenteiles 18 sind wandungsaußenseitig der Breitseitenwände 16 nach außen abragende Schwenkbolzen 19 angeformt, die in entsprechend positionierte und dimensionierte Vertiefungen 20 oder Durchbrechungen im Bereich der aufeinander zu weisenden Innenseiten der Breitseitenwände 8 des feststehenden Gehäuseteiles 2' eingreifen. Die sich hieraus ergebende Schwenkachse y des Gehäuseteiles 2' erstreckt sich entsprechend bevorzugt parallel zur Schwenkachse x der Kappe 3.

In dem insgesamt eine Aufnahme A bildenden, schwenkbaren Gehäuseteil 2' ist ein Schieberraum 21 ausgeformt. Dieser ist mit Bezug auf einen Vertikalquerschnitt im Wesentlichen rechteckig bis hin zu quadratisch gebildet. Der Schieberraum 21 weist einen sich zwischen den Innenseiten der Breitseitenwände 16 erstreckenden Schieberraumboden 22 auf. Dieser ist - zugewandt der Schmalseitenwand 17 - mit dieser verbunden.

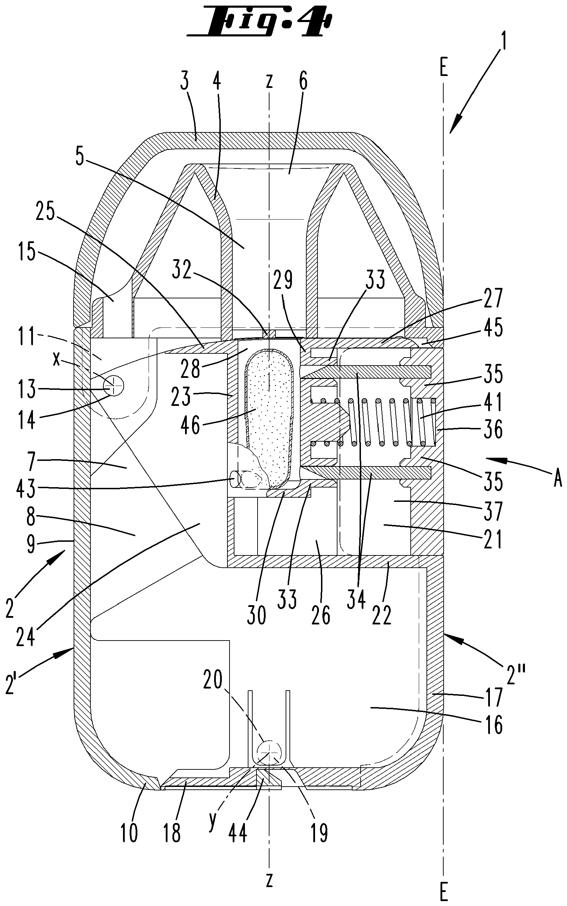

Von diesem Anbindungsbereich an der Schmalseitenwand 17 erstreckt sich der Schieberraumboden 22 bevorzugt unter paralleler Ausrichtung zu dem Boden des Gehäuses 2 beziehungsweise unter Querausrichtung einer die Vorrichtung 1 bevorzugt zentral durchsetzenden Körperachse z in den Raum 7. Endseitig ist an dem Schieberraumboden 22 eine in Grundstellung der Vorrichtung 1 senkrecht ausgerichtete Schieberraumwandung 23 ausgeformt. Deren nach vertikal oben weisende Randkante stößt in der Grundstellung gemäß Fig. 4 gegen einen zugeordneten unteren Randabschnitt des Mundstückkanals 5.

Der so ausgebildete Schieberraum 21 ist im Wesentlichen nach oben geöffnet in Richtung auf den Mundstückkanal 5 sowie zur Seite geöffnet, hierbei die Schmalseitenwand 17 durchbrechend.

Auf der dem Schieberraum 21 abgewandten Seite sind an der Schieberraumwandung 23 jeweils die Innenseite der zugewandten Breitseitenwände 8 des feststehenden Gehäuseteiles 2' flankierende, in horizontaler Projektion auf eine Breitseitenwand 8 betrachtet kreissegmentartige Flügelabschnitte 24 angeformt. Der Radius dieser kreissegmentartigen Flügelabschnitte 24 entspricht im Wesentlichen dem Vertikalerstreckungsmaß der Schieberraumwandung 23, wobei eine Radiuslinie etwa vom Übergangsbereich von Schieberraumwandung 23 in den Schieberraumboden 22 ausgeht. Entsprechend läuft die im Wesentlichen nach oben gerichtete kreislinienabschnittförmige Randkante eines jeden Flügelabschnittes 24 bevorzugt stufenlos in die nach oben weisende Randkante der Schieberraumwandung 23 ein. In dem Übergangsbereich von Flügelabschnitt 24 zur Schieberraumwandung 23 ist ein, oberseitig die Krümmung des Flügelabschnittes 24 aufnehmder, in Richtung auf die Innenseite der Schmalseitenwand 9 des feststehenden Gehäuseteils 2' gerichteter Deckenabschnitt 25 angeformt.

Bevorzugt einstückig und materialeinheitlich angeformt sind innerhalb des Schieberraumes 21 auf dem Schieberraumboden 22, parallel zu den Breitseitenwänden 16 verlaufende Führungswandungen 26 angeordnet. Diese sind jeweils zu der Ebene der zugeordneten Breitseitenwand 16 versetzt vorgesehen, insbesondere beidseitig gleichmäßig aufeinander zu versetzt, so dass sich zwischen der Führungswandung 26 und der zugewandten Breitseitenwand 8 des feststehenden Gehäuseteiles 2 in der Grundstellung der Vorrichtung 1 eine schlitzförmige Führung ergibt.

Die Führungswandungen 26 tragen eine bevorzugt parallel zum Schieberraumboden 22 verlaufende Decke 27, welche in den vorbeschriebenen Deckenabschnitt 25 übergeht.

Unterseitig der Decke 27 ist eine in einem Horizontalquerschnitt betrachtet kreisscheibenförmige Kapselkammer 28 angeordnet. Deren umlaufende Begrenzungswandung ist teilweise durch die Schieberraumwandung 23 gebildet, zum weiteren Teil durch eine von der Decke 27 abgehängte Wandung 29.

Die Kapselkammer 28 öffnet sich nach oben unter Durchsetzung der Decke 27.

Nach unten ist die Kapselkammer 28 begrenzt durch einen geschlossenen Kammerboden 30, welcher sich in der dargestellten Ausführungsform bevorzugt mit vertikalem Abstand zu dem Schieberraumboden 22 erstreckt.

Der Durchmesser der Kapselkammer 28 ist bevorzugt so gewählt, dass dessen Durchmesser etwa dem 1,2- bis 1,4-Fachen des Kapseldurchmessers entspricht, so dass eine in die Kapselkammer 28 eingeführte Kapsel 31 auf dem Kammerboden 30 stehend lose einliegt. Die vertikale Höhe der Kapselkammer 28 entspricht weiter bevorzugt etwa dem 1,05- bis 1,2-Fachen der Kapsellänge.

Die Kapselkammer 28 ist weiter bevorzugt - zumindest in der Grundstellung - in axialer Verlängerung zu dem Mundstückkanal 5 angeordnet, wobei fußseitig des Mundstückkanals 5, quasi in der Trennungsebene zwischen dem Mundstückkanal 5 und der Kapselkammer 28 ein Rückhaltegitter 32 vorgesehen ist.

Die Wandung 29 der Kapselkammer 28 weist zwei vertikal übereinander liegende, in Richtung auf die Ebene der Schmalseitenwand 17 gerichtete Führungsbuchsen 33 auf. Die die Führungsbuchsen 33 entsprechend durchsetzenden Bohrungen öffnen sich sowohl in Richtung auf die Schmalseitenwand 17 als auch in Richtung auf das Kapselkammerinnere. Die Buchsen 33 dienen zur Führung von Nadeln 34, welche mit ihren Nadelspitzen in Richtung auf das Kapselkammerinnere gewandt sind.

Die Nadeln 34 sind in Nadellagern 35 gefasst. Diese Nadellager 35 sind an einer abgefederten Drucktaste 36 ausgebildet.

Die Drucktaste 36 weist eine der Tiefe der Schmalseitenwand 17 entsprechende Tiefe auf sowie eine vertikale Höhe, die der freien vertikalen Höhe des Schieberraumes 21 entspricht.

An der Drucktaste 36 sind innenseitig, in den Schieberraum 21 einragend, Gegenführungswandungen 37 angeformt. Diese sind in Tiefenrichtung der Vorrichtung 1 betrachtet so zueinander beabstandet, dass diese in den schlitzförmigen Raum zwischen Führungswandung 26 und Breitseitenwand 8 des feststehenden Gehäuseteiles 2 eintauchen.

Zwischen den aufeinander zu weisenden Flächen von Gegenführungswandungen 37 und Führungswandung 26 ist eine Formschlussführung 38 ausgebildet. Hierzu sind wandungsinnenseitig, entsprechend nach innen und aufeinander zu gewandt, an den Gegenführungswandungen 37 Zapfen 39 angeformt. Diese greifen in wandungsaußenseitig der Führungswandungen 26 ausgebildete, nutartige Vertiefungen 40.

Die Federbelastung der Drucktaste 36 ist erreicht durch eine zwischen Drucktaste 36 und Wandung 29 gelagerte Druckfeder 41. Diese belastet entsprechend die Drucktaste 36 nach außen in Richtung auf die Schmalseitenwand 17, wobei diese Verlagerungsrichtung der Drucktaste 36 anschlagbegrenzt ist zufolge eines die Vertiefung 40 für den Zapfen 39 querenden Stegs 42.

Die Anschlagbegrenzung in der Formschlussführung 38 ist hierbei so gewählt, dass in der druckfederbelasteten Grundstellung eine nach außen gerichtete Fläche der Drucktaste 36 in der Schmalseitenebene E liegt, hierbei entsprechend eine Verlängerung beziehungsweise einen Teil der Schmalseitenwand 17 insgesamt ausbildet.

Dem Kammerboden 30 zugewandt ist in der Schieberraumwandung 23 eine sich zum Raum 7 hin öffnende Durchströmöffnung 43 ausgebildet. Diese ist weiter bevorzugt so angeordnet, dass ein durch die Öffnung 43 eingesaugter Luftstrom im Wesentlichen tangential in die Kapselkammer 28 eintritt.

Durch die vorbeschriebene Ausgestaltung der Vorrichtung liegt diese in der Nichtbenutzungsstellung gemäß Fig. 1, d.h. weiter in einer Verwahrstellung derselben, in einer insgesamt geschlossenen Bauform vor, dies weiter bevorzugt bezüglich aller Flächenebenen insbesondere ohne Vorsprünge. Dies bietet weiter einen erhöhten Komfort insbesondere beim Tragen der Vorrichtung 1 in einer körpernahen Tasche. Auch ist hierdurch die Drucktaste 36 zufolge Einfügen derselben in die Schmalseitenwandebene E nicht ohne Weiteres zufällig betätigbar.

In der Kappenverschlussstellung gemäß Fig. 1 ist neben dem Mundstück 4 beziehungsweise dem Mundstückkanal 5 und der Lufteinlassöffnung 15 auch die Kapselkammer 28 vor Eindringen von Schmutz oder dergleichen geschützt.

Zur Vorbereitung eines Inhalationsvorganges wird das Gehäuseteil 2" unter Halten der Vorrichtung 1 im Bereich des festen Gehäuseteiles 2' um die Achse y nach außen geschwenkt (vergleiche Fig. 3). Diese ausgeschwenkte Stellung ist bevorzugt anschlagbegrenzt, dies insbesondere zufolge Anschlagen einer Randkante des Bodenteiles 18 des schwenkbaren Gehäuseteiles 2" an einem wandungsinnenseitig an zumindest einer Breitseitenwand 8 angeformten Anschlagbock 44.

In dieser Schwenkstellung des Gehäuseteiles 2" überdeckt der Deckenabschnitt 25 den freien Zugriff auf den Raum 7, wobei weiter die angeformten Flügelabschnitte 24 eine Führung des schwenkbaren Gehäuseteiles 2" in dem feststehenden Gehäuseteil 2' bieten.

Das Erfassen des schwenkbaren Gehäuseteiles 2" ist erleichtert durch in Richtung auf die Schmalseitenwand 17 hin offene, in den Breitseitenwänden 8 des feststehenden Gehäuseteiles 2' ausgebildete, fensterartige Ausbrüche 45. Entsprechend kann das schwenkbare Gehäuseteil 2" durch diese fensterartigen Ausbrüche 45 beidseitig mit zwei Fingern erfasst werden. Die Höhe der fensterartigen Ausbrüche 45 entspricht hierbei bevorzugt der Höhe der Drucktaste 36, wobei weiter die quer hierzu betrachtete Breite eines jeden Ausbruches 45 ausgehend von der der Schmalseitenwand 17 zugewandten Randkante der Breitseitenwand 8 angepasst ist an den möglichen Verlagerungsweg der Drucktaste 36.

In der ausgeschwenkten Stellung des Gehäuseteiles 2" ist der Einsteckquerschnitt der Kapselkammer 28 in spitzwinkliger Anschlagstellung gestoppt, dies bevorzugt einen spitzen Winkel α von 30 bis 45 Grad zu der Körperachse z einnehmend (dies weiter mit Bezug auf eine die Kapselkammer 28 zentral durchsetzende Längsachse).

In dieser Stellung wird die Kapsel 31 in die Kapselkammer 28 eingeführt, wonach das Gehäuseteil 2" beziehungsweise die Aufnahme A wieder zurück in die, in das Gehäuseteil 2' zumindest teilweise eintauchende Stellung zurückverlagert wird. Es ergibt sich hiernach eine Inhalations-Bereitschaftsstellung gemäß Fig. 4.

Um den Inhalationsvorgang durchzuführen, wird vor oder auch nach einem Abschwenken der Kappe 3 zum Freilegen des Mundstückes 4 die Wandung der Kapsel 31 durchstoßen, dies zufolge entsprechender linearer Verlagerung der Drucktaste 36 entgegen der Kraft der Druckfeder 41. Die hierbei mitverlagerten Nadeln 34 durchstechen entsprechend die Kapselwandung. Die Nadeln 34 durchstoßen im Zuge deren Verlagerung in Richtung auf die Kapselkammer 28 die Kapselwandung sowohl im Zuge des Eintauchens der Nadelspitzen in das Kapselinnere als auch beim Austauchen aus dem Kapselinneren nach außen. Die Anordnung der Nadeln 34 ist hierbei bevorzugt so gewählt, dass diese die Kapsel 31 jeweils etwa im Bereich des Überganges vom zylindrischen Mittenabschnitt zu den Kappenendabschnitten durchstoßen.

Mit Fortfall der Beaufschlagung der Drucktaste 36 durch den Benutzer wird dies selbsttätig zurückverlagert in die anschlagbegrenzte Grundstellung.

Zum Inhalieren wird das Mundstück 4 von den Lippen umschlossen, wonach zufolge Einatmen eine Luftströmung (Pfeil c) erzeugt wird. Diese tritt über die Lufteinlassöffnung 15 in den Raum 7 ein und wird durch die Durchströmöffnung 43 in die Kapselkammer 28 gesogen. Die zufolge des bevorzugten tangentialen Einlasses die Kapselkammer 28 wirbelartig durchsetzende Luft strömt zugleich durch die durchgestochene Kapsel 31 zum Ausräumen der hier bevorrateten Substanz 46. Das Luft-Substanz-Gemisch wird über den Mundstückausgang 6 eingeatmet, wobei die Kapsel 31 beziehungsweise die Kapselwandung durch das Gitter 32 an einem Mitreißen in der Luftströmung gehindert wird.

In vorteilhafter Weise ist insbesondere in der aufgeschwenkten Stellung der Aufnahme A beziehungsweise des Gehäuseteiles 2" eine gute Durchlüftung der vom Saugstrom durchsetzten Bereiche gegeben, so insbesondere bezüglich der Lufteinlassöffnung 15 und/oder des Mundstückkanals 5 und/oder des Raumes 7.

**Bezugszeichenliste:**

| | | | |
|---|---|---|---|
| 1 | Vorrichtung | 25 | Deckenabschnitt |
| 2 | Gehäuse | 26 | Führungswandung |
| 2' | Feststehendes Gehäuseteil | 27 | Decke |
| 2" | Schwenkbares Gehäuseteil | 28 | Kapselkammer |
| 3 | Kappe | 29 | Wandung |
| 4 | Mundstück | 30 | Kammerboden |
| 5 | Mundstückkanal | 31 | Kapsel |
| 6 | Mundstückausgang | 32 | Rückhaltegitter |
| 7 | Raum | 33 | Führungsbuchse |
| 8 | Breitseitenwand | 34 | Nadel |
| 9 | Schmalseitenwand | 35 | Nadellager |
| 10 | Bodenteilabschnitt | 36 | Drucktaste |
| 11 | Lasche | 37 | Gegenführungswandung |
| 12 | Vertiefung | 38 | Formschlussführung |
| 13 | Zapfen | 39 | Zapfen |
| 14 | Zapfenaufnahme | 40 | Vertiefung |
| 15 | Lufteinlassöffnung | 41 | Druckfeder |
| 16 | Breitseitenwand | 42 | Steg |
| 17 | Schmalseitenwand | 43 | Durchströmöffnung |
| 18 | Bodenteil | 44 | Anschlagbock |
| 19 | Schwenkbolzen | 45 | Ausbruch |
| 20 | Vertiefung | 46 | Substanz |
| 21 | Schieberraum | | |
| 22 | Schieberraumboden | c | Pfeil |
| 23 | Schieberraumwandung | x | Schwenkachse |
| 24 | Flügelabschnitt | y | Schwenkachse |
| Z | Körperachse | | |
| | | | |
| A | Aufnahme | | |
| E | Schmalseitenwandebene | | |
| | | | |
| α | Winkel | | |

## Patentansprüche

1. Vorrichtung (1) zum Inhalieren pulverförmiger Substanzen (46), insbesondere medizinischer Art, enthalten in Kapseln (31), die mittels einer in einem Flachgehäuse (2) verlagerbaren Aufnahme (A) in eine Entleerungsstellung bringbar sind, wobei die Vorrichtung (1) einen Breiten-Höhen-Verhältnis von 1 : 2 aufweist und eine senkrecht zur Höhenerstreckung betrachtete Tiefe aufweist, die etwa dem halben Breitenerstreckungsmaß des Flachgehäuses (2) entspricht, wobei weiter in der Entleerungsposition eine Kapselwandung mittels verschieblicher Nadeln (34) durchstechbar ist zwecks Leersaugen des Kapselinhaltes durch einen Mundstückkanal (5) welcher in axialer Verlängerung einer Kapselkammer (28) verläuft und wobei die Aufnahme (A) als um eine Achse (y) begrenzt ausschwenkbarer Teil einer Schmalseitenwand (17) des Flachgehäuses (2) ausgebildet ist, **dadurch gekennzeichnet, dass** ein Teilbereich einer Schmalseitenwandebene (E) als abgefederte Drucktaste (36) für Nadellager (35), in welchen die Nadeln (34) gefasst sind, ausgebildet ist, derart, dass sich der Teilbereich bis maximal in die Ebene (E) der Schmalseitenwand (17) zurückstellt und dass die Schmalseitenwand (17) mitsamt einem nahezu halben, kastenförmigen Gehäuseteil (2") ausschwenkt, einschließlich eines Bodenteils (18), welches den Schwenkbolzen (19) besitzt, und dass die Drucktaste (36) in dem ausschwenkbaren Gehäuseteil (2") anschlagbegrenzt geführt ist, wobei weiter auch ein feststehender Gehäuseteil (2') einen im Wesentlichen halben, kastenförmigen Gehäuseabschnitt bildet.

2. Vorrichtung nach Anspruch 1, **dadurch gekennzeichnet, dass** der Einsteckquerschnitt der Kapselkammer (28) in der Öffnungsstellung der Aufnahme (A) in spitzwinkeliger Anschlagstellung gestoppt ist.

3. Vorrichtung nach einem der vorhergehenden Ansprüche, **gekennzeichnet durch** eine Formschlussführung (38) der abgefederten Drucktaste (36) in eine Stellung, in der die Drucktaste (36) mit dem restlichen Bereich der Schmalseitenwand (17) in einer Ebene (E) liegt.

4. Vorrichtung nach einem der vorhergehenden Ansprüche, **dadurch gekennzeichnet, dass** in dem schwenkbaren Gehäuseteil (2") ein Schieberraum (21) ausgebildet ist und an der Drucktaste (36) innenseitig, in dem Schieberraum (21) einragend, gegen Führungswandungen (37) ausgeführt sind.

## Claims

1. Device (1) for inhaling pulverulent substances (46), in particular of a medicinal type, contained in capsules (31) which can be moved into an emptying position by means of a receptacle (A) which is movable in a flat housing (2), the device (1) having a width-height ratio of 1 : 2 and a depth that corresponds to approximately half the size of the width extension of the flat housing (2) when viewed perpendicularly to the height extension thereof, a capsule wall further being pierceable in the emptying position by means of displaceable needles (34) for the purposes of evacuating the capsule contents through a mouthpiece duct (5) extending as an axial extension of a capsule chamber (28), and the receptacle (A) being formed as a part of a narrow side wall (17) of the flat housing (2) that can pivot out in a limited manner about an axis (y), **characterised in that** a portion of a narrow side wall plane (E) is formed as a spring-mounted button (36) for needle bearings (35), in which the needles (34) are mounted, such that the portion is set back at most as far as into the plane (E) of the narrow side wall (17), such that the narrow side wall (17) pivots out together with approximately half a box-shaped housing part (2"), including a bottom part (18) which has the pivot pin (19), and such that the button (36) is guided, in a manner limited by a stop, in the housing part (2") which can be pivoted outwards, a stationary housing part (2') further forming substantially half a box-shaped housing portion.

2. Device according to claim 1, **characterised in that** the insert cross section of the capsule chamber (28) is stopped in an acute-angled stop position when the receptacle (A) is in the open position.

3. Device according to either of the preceding claims, **characterised by** an interlocking guidance (38) of the spring-mounted button (36) into a position in which the button (36) is located in a plane (E) together with the rest of the region of the narrow side wall (17).

4. Device according to any of the preceding claims, **characterised in that** a slide space (21) is formed in the pivotable housing part (2") and counter guide walls (37) are formed on the inside of the button (36), which walls project into the slide space (21).

## Revendications

1. Dispositif (1) pour l'inhalation de substances pulvérulentes (46), en particulier de type médical, contenues dans des capsules (31) lesquelles sont susceptibles d'être amenées dans une position de vidage au moyen d'un réceptacle (A) déplaçable dans un boîtier plat (2), dans lequel le dispositif (1) présente un rapport largeur-hauteur de 1:2 et une profondeur, considérée perpendiculairement à l'extension selon la hauteur, qui correspond environ à la moitié de la dimension d'extension selon la largeur du boîtier plat (2), dans lequel en outre, dans la position de vidage, une paroi de capsule est susceptible d'être transpercée au moyen d'aiguilles déplaçables (34) aux fins de vidage du contenu de la capsule par aspiration au travers d'un canal d'embout (5) lequel s'étend dans le prolongement axial d'une chambre à capsule (28) et dans lequel le réceptacle (A) est réalisé en tant que partie d'une paroi de côté étroit (17) du boîtier plat (2) qui est susceptible de pivoter vers l'extérieur de manière limitée autour d'un axe (y), **caractérisé en ce qu'**une partie d'un plan de paroi de côté étroit (E) est réalisée en tant que bouton-poussoir (36) contraint élastiquement pour paliers d'aiguilles (35) dans lesquels les aiguilles (34) sont maintenues, de manière à ce que la partie revienne en arrière au maximum jusque dans le plan (E) de la paroi de côté étroit (17) et que la paroi de côté étroit (17) ensemble avec approximativement la moitié d'une partie de boîtier en forme de boîte (2") pivote vers l'extérieur, y inclus une partie de fond (18) qui comprend l'axe de pivotement (19), et que le bouton-poussoir (36) est guidé de manière limitée par butée dans la partie de boîtier pivotante vers l'extérieur (2"), dans lequel en outre une partie de boîtier fixe (2') forme aussi sensiblement une moitié de section de boîtier en forme de boite.

2. Dispositif selon la revendication 1, **caractérisé en ce que** dans la position d'ouverture du réceptacle (A), la section d'insertion de la chambre de capsule (28) est arrêtée avec un angle aigu en position de butée.

3. Dispositif selon l'une des revendications précédentes, **caractérisé par** un guidage par coopération de forme (38) du bouton-poussoir contraint élastiquement (36) dans une position dans laquelle le bouton-poussoir (36) se situe dans un plan (E) avec le reste de la région de la paroi de côté étroit (17).

4. Dispositif selon l'une des revendications précédentes, **caractérisé en ce qu'**un espace de coulissement (21) est formé dans la partie de boîtier pivotante (2") et que des contre-parois de guidage (37) sont réalisées du côté intérieur sur le bouton-poussoir (36) en faisant saillie dans l'espace de coulissement (21).
